**Europäisches Patentamt**

**European Patent Office**

**Office européen des brevets**

(11) Veröffentlichungsnummer: **0 181 586**

A2

(12) **EUROPÄISCHE PATENTANMELDUNG**

(21) Anmeldenummer: 85113973.3

(22) Anmeldetag: 26.04.83

(51) Int. Cl.⁴: **A 61 F 2/36**

(30) Priorität: 03.05.82 DE 3216539

(43) Veröffentlichungstag der Anmeldung:
21.05.86 Patentblatt 86/21

(84) Benannte Vertragsstaaten:
CH DE FR GB IT LI SE

(60) Veröffentlichungsnummer der früheren
Anmeldung nach Art. 76 EPÜ: 0 093 378

(71) Anmelder: Waldemar Link GmbH & Co
Barkhausenweg 10
D-2000 Hamburg 63(DE)

(72) Erfinder: Keller, Arnold
An der Naherfurth 5
D-2061 Keyhude(DE)

(74) Vertreter: Glawe, Delfs, Moll & Partner Patentanwälte
Postfach 26 01 62 Liebherrstrasse 20
D-8000 München 26(DE)

(54) **Femorale Hüftfelenkprothese.**

(57) Eine femorale Hüftgelenkendoprothese mit einem zur zementfreien Verankerung im Femur (1) vorgesehenen Schaft (6), dessen proximaler Endabschnitt zur weitgehenden Füllung des spongiösen Teils des Femurs in dessen trochanterem Bereich (14) gegenüber seinem distalen Abschnitt (12) stark verdickt ist, zeichnet sich dadurch aus, daß die Verdickung in Form einer Mehrzahl von einem schlanken Schaftkern (17) auf dessen dorsaler und ventraler Seite vorspringenden Längsrippen (15) gebildet ist, die der medialen Seite ein Breitfläche zukehren und eine Vielzahl von zum Rippenrücken (18) hin im wesentlichen geschlossenen Durchbrechungen (20) aufweisen. Dadurch werden die Bedingungen für die Kraftübertragung im proximalen Endabschnitt des Prothesenschafts auf das Knochengewebe verbessert, wobei gleichzeitig die Elastizität des Schafts günstig beeinflußt werden kann.

./...

EP 0 181 586 A2

Fig. 1

Fig. 2

GLAWE, DELFS, MOLL & PARTNER

PATENTANWÄLTE
ZUGELASSENE VERTRETER BEIM EUROPÄISCHEN PATENTAMT

RICHARD GLAWE
DR · ING

KLAUS DELFS
DIPL · ING

ULRICH MENGDEHL
DIPL · CHEM. DR. RER. NAT

WALTER MOLL
DIPL · PHYS DR RER. NAT
ÖFF. BEST. DOLMETSCHER

HEINRICH NIEBUHR
DIPL · PHYS. DR. PHIL. HABIL.

8000 MÜNCHEN 26
POSTFACH 182
LIEBHERRSTR. 20
TEL. (089) 22 65 48
TELEX 5 22 505 SPEZ
TELECOPIER (0 89) 22 39 38

2000 HAMBURG 13
POSTFACH 25 70
ROTHENBAUM-
CHAUSSEE 58
TEL. (040) 4 10 20 08
TELEX 21 29 21 SPEZ

HAMBURG

Waldemar Link GmbH & Co

Hamburg

---

Femorale Hüftgelenkprothese

---

p 10761/83

D/fi

Beschreibung

Die Erfindung betrifft eine femorale Hüftgelenkprothese mit
einem zur zementfreien Verankerung im Femur vorgesehenen
Schaft, dessen proximaler Endabschnitt zur weitgehenden
Füllung des spongiösen Teils des Femurs in dessen trochanterem Bereich gegenüber seinem distalen Abschnitt stark
verdickt ist.

Im Bereich des Femurkörpers distal von den Trochantern ist
die harte Außenschicht des Knochens, die Corticalis, verhältnismäßig dick und der Markkanal dünn. In diesem Bereich
kann ein Prothesenschaft verhältnismäßig sicher verankert
werden, weil eine direkte oder über Knochenzement vermittelte
Kraftübertragung von der Prothese auf die Corticalis möglich
ist. Im proximalen Femurbereich ist die sich trichterförmig erweiternde Corticalis sehr dünn und wird der Knochenquerschnitt im wesentlichen von einem feinlamellaren Trajektoriensystem des spongiösen Knochengewebes gebildet, das bei der Kraftübertragung von der Prothese her nur wesentlich geringeren spezifischen Belastungen ausgesetzt werden darf. Dieser Bereich, der für die Zwecke der vorliegenden Beschreibung als der trochantere Femurbereich bezeichnet wird, liegt etwa oberhalb der unteren Begrenzung des kleineren Trochanters. Wird die Prothese in diesem Bereich unter Vermittlung von Knochenzement verankert, so kann durch die Ausbildung der vor dem Einsetzen der Prothese ausgeraspelten Knochenhöhlung und der daraus resultierenden Form des Zementkörpers die Form und Größe der kraftübertragenden Fläche hinreichend bemessen werden. Wenn hingegen die Verankerung ohne Knochenzement erfolgt, muß der Prothesenschaft als solcher eine hinreichend große Kraftübertragungsfläche zur Verfügung stellen und daher in seinem proximalen Bereich stark verdickt sein, was in diesem Bereich zu großer Steifigkeit des Schafts führt. Angestrebt wird jedoch eine Annäherung der Schaftelastizität an diejenige des Knochens (Z.Orthop. 1979, S. 481), da nur so eine gleichmäßige Kraftübertragung möglich ist. Andernfalls kann es zu Lockerungserscheinungen wegen örtlicher Knochenrückbildung aufgrund von Über- bzw. Entlastung des Knochengewebes kommen.

Bei der aus der US-PS 2 719 522 bekannten Femurprothese trägt der trichterförmig verdickte, proximale Endabschnitt des Prothesenschafts auf seiner medialen und lateralen Seite eine Vielzahl von flachen Nuten und Rippen, die wahrscheinlich der Drehsicherung des Prothesenschafts im Knochen dienen sollen. Die vom Prothesenschaft auf den Knochen zu übertragenden Kräfte werden dadurch weder verringert, noch wird ihre Übertragung erleichtert.

Nach dem DE-GM 81 24 912 sind die ventralen und dorsalen Flächen des Prothesenschafts mit flachen Noppen besetzt, die bei zementfreier Verankerung eine Verkeilung des Prothesenschafts im Knochen und seine dauerhafte Festlegung bezwecken. Da jedoch ein Teil der von der Gewichtsbelastung durch den menschlichen Körper herrührenden Kräfte im Trochanterbereich nach der medialen Seite hin durch Druck und nach der lateralen Seite hin durch Zug übertragen werden muß, kann nicht erwartet werden, daß diese Noppen die Kraftübertragungsverhältnisse im Trochanterbereich verbessern, da ihre medial gerichteten Flächenanteile im Vergleich mit der Größe der medialen Schaftstirn, die für die Übertragung der Druckkräfte hauptsächlich verantwortlich ist, zu gering sind.

Nach der CH-PS 622 423 ist der proximale Endabschnitt des Prothesenschafts mit fischgrätartig verlaufenden Erhöhungen versehen, die beim Eintreiben des Schafts in die operativ vorbereitete Knochenhöhlung die Verteilung des verdrängten, spongiösen Knochengewebes verbessern und damit die Verankerung des Schaftes durch rasches Einwachsen beschleunigen sollen. Eine Rolle bei der Übertragung der im proximalen Endabschnitt nach medial gerichteten Kräfte ist ihnen offenbar nicht zugedacht, wozu sie aufgrund ihrer Form auch kaum geeignet sind.

Die DE-OS 28 39 092 zeigt einen Prothesenschaft, der als ein mit Längsrippen versehener, in den Knochenkanal eintreibarer Nagel ausgebildet ist, der zumindest in seinem distalen Abschnitt kreuzförmige Gestalt hat. Im proximalen Endabschnitt findet in der Projektion nach medial keine Verdickung statt; es steht daher zur Übertragung der nach medial gerichteten

Glawe, Delfs, Moll & Partner - p 10761/83 - Seite 4

Kräfte keine größere Oberfläche als im distalen Schaftteil zur Verfügung. Vergrößert wird lediglich die nach medial weisende Rippe zu Lasten der lateralen Rippe, wodurch ein Abstützeffekt zur Verstärkung des Schafts als solchem erzielt werden soll; die Kraftübertragung auf den Knochen wird dadurch nicht verbessert - im Gegenteil bewirkt die mediale Rippe eine tiefe Aufteilung des medial vom proximalen Schaftabschnitt gelegenen, hochbelasteten Knochengewebes und damit eine Schwächung desselben.

Nagelförmige, im proximalen Endabschnitt nicht wesentlich verdickte Prothesenschäfte zeigen ferner die US-PS 3 067 740 und 3 740 769.

Dieser        Stand der Technik läßt keine Mittel erkennen, die die Kraftübertragung vom proximalen Endabschnitt des Prothesenschafts nach medial auf den Knochen im Sinne geringerer spezifischer Belastungen und höherer Schaftelastizität verbessern könnten. Der Erfindung liegt daher die Aufgabe zugrunde, die Kraftübertragungsverhältnisse im poximalen Endabschnitt des Prothesenschafts zu verbessern.

Die erfindungsgemäße Lösung besteht darin, daß die Verdickung des Prothesenschafts in Form einer Mehrzahl von von einem schlanken Schaftkern auf dessen dorsaler und ventraler Seite vorspringenden Längsrippen ausgebildet ist, die der medialen Seite eine Breitfläche  zukehren und eine Vielzahl von zum Rippenrücken hin im wesentlichen geschlossenen Durchbrechungen aufweisen.

Über die der medialen Seite zugekehrte Breitfläche können die Rippen zusätzlichlich zur Schaftoberfläche nach medial gerichtete Kräfte übertragen und verringern dadurch die spezifische Belastung des Knochengewebes in dieser Richtung.

Die Durchbrechungen der Rippen verringern die Wirksamkeit
der Kraftübertragung nicht, weil die einzelnen Durchbrechungen
in Folge ihrer Vielzahl klein sind und das in sie hineinwachsende Gewebe daher festhalten und es an der Kraftübertragung beteiligen. Nicht nur die am weitesten medial gelegene Rippe auf der dorsalen bzw. ventralen Seite des
Schafts beteiligt sich an der Kraftübertragung nach medial;
vielmehr gilt dies auch für die folgende Rippe bzw. folgenden
Rippen, weil diese mit ihren nach medial gerichteten Breitflächen auf das zwischen die Rippen einwachsende Knochengewebe kraftübertragend einwirken. Von diesem aus können die
Kräfte unproblematisch an die den Prothesenschaft umgebenden,
härteren Querschnittsteile des Knochens weitergegeben werden,
weil die Rippen keine Aufteilung des Knochengewebes bewirken;
denn das zwischen den Rippe befindliche Knochengewebe steht
über die Vielzahl der Rippendurchbrechungen mit dem übrigen
Knochengewebe abstützend in Verbindung. Die Durchbrechungen
bezwecken zusätzlich zu ihrer Aufgabe, die beiderseits einer
Rippe liegenden Knochenbereiche abstützend zu verbinden, die
Steifheit der Rippen gegenüber Biegebeanspruchung des
Schaftes zu verringern, um dadurch seine Elastizität zu
erhöhen. Ihre Anordnung kann nach bekannten statischen
Grundsätzen so gewählt sein, daß diesem Ziel am besten entsprochen wird. Die Verringerung der Rippensteifheit gelingt
weitgehend, wenn die Öffnungen zum Rippenrücken hin (das ist
die Fläche der Rippe, die vom Schaft wegweist) zumindest
teilweise geöffnet sind. Damit die Rippen jedoch nicht
nur mit ihren Breitseiten und mit ihren Rücken Druckkräfte
auf das Gewebe übertragen können sondern auch im Bereich
ihrer Öffnungen Zugkräfte, dürfen nach der Erfindung die
Durchbrechungen zum Rippenrücken hin nicht vollständig geöffnet sein. Sie sind im Sinne der Erfindung im wesentlichen
geschlossen, wenn ein wesentlicher Teil ihrer näher dem Rippenrücken gelegenen Begrenzungsfläche geschlossen ist.
Beispielsweise ist es möglich, alle oder einzelne
Durchbrechungen durch einen dünnen Schlitz zum Rippenrücken hin zu öffnen und dadurch die Rippensteifigkeit zu
verringen, ohne daß dadurch der geschlossene Charakter dieser
Durchbrechungen verlorengeht.

Zweckmäßigerweise ist auch auf der lateralen Schaftkernseite im proximalen Endabschnitt des Prothesenschafts
mindestens eine Rippe mit zum Rippenrücken hin im wesentlichen geschlossene Durchbrechungen vorgesehen, durch die
bewirkt werden soll, daß der laterale Knochenbereich beim
größeren Trochanter durch Übertragung von Zugkräften von
der Prothese auf diesen Knochenbereich an der Kraftübertragung beteiligt wird. Dadurch wird einerseits der mediale
Knochenbereich entlastet und andererseits der laterale
Knochenbereich vor Rückbildungserscheinungen, die auf zu
starke Kraftentlastung zurückzuführen sind, weitgehend geschützt.

Die mediale Seite des proximalen Endabschnitts des Prothesenschafts kann nach der Erfindung im wesentlichen rippenfrei
sein, um die Einheitlichkeit des hoch belasteten, medial
gelegenen Knochenbereichs nicht zu stören. Kleinere Erhöhungen oder Vertiefungen werden von dieser Überlegung nicht
betroffen.

Die Mittelachsen von einander an der ventralen oder dorsalen
Seite des Schafts benachbarten Rippen schließen zweckmäßigerweise            im Querschnitt einen Winkel von nicht mehr
als 30° miteinander ein und sind weiter vorzugsweise sogar
etwa parallel zueinander gerichtet, um dadurch einen um so innigeren Verbund des in den Zwischenraum einwachsenden
Knochengewebes mit den Rippen zu erzielen. Dies schließt
nicht aus, daß die Rippen am Rand im Querschnitt abgerundet sind, um einen harmonischen Kräfteverlauf im Knochengewebe zu erreichen. Dem gleichen Zweck dient es, wenn nach
der Erfindung einander benachbarte Rippen an der dorsalen
bzw. ventralen Seite des Schafts einen Zwischenraum einschließen, dessen auf den trochanteren Femurbereich bezogene
mittlere Querschnittstiefe mindestens etwa seiner Weite
gleicht. Dadurch wird nämlich die Verzahnung des Schafts
mit dem Knochengewebe verbessert.

Zweckmäßigerweise sind auf der dorsalen und der ventralen
Seite des proximalen Endabschnitts des Schafts jeweils
mindestens zwei Rippen vorgesehen. Insbesondere im endnahen,
dickeren Schaftbereich können auch mehr, beispielsweise
drei, Rippen vorgesehen sein.

Die lichten Querabmessungen der Durchbrechungen sind vorteilhafterweise nicht größer als etwa 5 mm, vorzugsweise nicht
mehr als 3 mm, um einen innigen Verbund mit dem Knochengewebe und insbesondere auch eine Übertragung von Zugkräften
von dem Schaft auf das Knochengewebe zu ermöglichen. Sie
können beispielsweise als Bohrungen mit rundem lichten Querschnitt ausgebildet sein.

Das Merkmal, daß die Rippen in Längsrichtung des Schaftes verlaufen, ist zum einen dadurch begründet, daß sie der medialen
Seite Breitflächen zukehren sollen. Zum anderen werden dadurch
Operation und Reoperation erleichtert.

Ein wesentliches Merkmal der Erfindung besteht darin, daß die
Querschnittshöhe der Rippen so groß bemessen ist, daß eine
Anpassung an unterschiedliche Femurabmessungen durch Abtragung vom Rippenrücken erzielbar ist. Zur Herstellung von Endoprothesen mit in die Markhöhle von Knochen einzusetzenden
Halteschäften von unterschiedlicher Dicke verfährt man daher
nach der Erfindung in solcher Weise, daß unter Verwendung
eines mit vorstehenden Rippen versehenen Halteschafts stets
gleicher Gestalt die unterschiedlichen Halteschäfte durch
Bearbeitung der Rippenrücken erzeugt werden. Auf diese Weise
läßt sich eine Vielzahl von Standardgrößen unter Verwendung
nur weniger Schaftrohlinge bereitstellen. Auch eine individuelle Anpassung an die durch Röntgenbild festgestellt Form
des die Prothese aufnehmenden Knochens ist denkbar.

Die Vorteile der Erfindung bestehen vornehmlich darin, daß
die Auflösung des Schaftquerschnitts in eine Mehrzahl von
Rippen zum einen eine weitgehend willkürliche Bemessung
des Widerstandsmoments und damit eine Anpassung der
Elastizität an diejenige des Knochens gestattet und daß
zum anderen die für den Verbund mit dem vorhandenen bzw.
einwachsenden Knochengewebe zur Verfügung stehende Oberfläche in der Hauptbelastungsrichtung vergrößert wird, so
daß ein fester Prothesensitz erzielt werden kann. Das in
die Zwischenräume der Rippen einwachsende, spongiöse Knochengewebe nimmt dank inniger Verankerung an der Kraftübertragung
und der Ernährung des Knochens teil.

Der Begriff Rippen beschränkt die Querschnittsgestalt der
so bezeichneten Prothesenteile nicht. Im allgemeinen setzt
er jedoch voraus, daß die Rippen zumindest auf einem wesentlichen Teil ihrer Länge eine Querschnittsgestalt mit größerer
Abmessung in ihrer von dem Schaftkern nach außen strebenden
Hauptrichtung als quer dazu aufweisen.

Das Merkmal, daß die Rippen in Längsrichtung verlaufen, besagt nicht, daß sie in dieser Richtung ununterbrochen ausgebildet sein müßten. Vielmehr können von außen nach innen geführte Vertiefungen, Schlitze oder Unterbrechungen zur Beeinflussung ihrer statischen Festigkeit vorteilhaft sein. Zur Beeinflussung ihrer Längsnachgiebigkeit kann auch ein wabenartiger Aufbau der Rippen bzw. ihrer Durchbrechungen vorgesehen sein. Der Rippenrücken kann mit Erhöhungen und Vertiefungen als Zahnung ausgebildet sein, die beim Eintreiben des Prothesenschafts sich selbst den Weg freischneidet.

Im Falle einer Reoperation kann es erforderlich sein, die zwischen den Rippen befindliche Knochensubstanz zu entfernen oder mindestens zu lösen. Da die Halsauflage dabei hinderlich sein kann, ist die Prothese nach einem weiteren Merkmal der Erfindung halsauflagenlos oder mit Einrichtungen zur lösbaren Befestigung einer Halsauflage ausgebildet.

Die Erfindung wird im folgenden näher unter Bezugnahme auf die Zeichnung erläutert, die vorteilhafte Ausführungsbeispiele veranschaulicht. Darin zeigen:

Fig. 1          einen Längsschnitt in der lateral-
                medial-Ebene eines Oberschenkelknochens
                mit eingesetzter Hüftgelenkprothese,

Fig. 2          einen Schnitt derselben Anordnung in
                der anterior-posterior-Ebene,

Fig. 3          eine der Fig. 2 entsprechende Schnitt-
                ansicht mit einer anderen Prothesen-
                form,

Fig. 4                    einen Horizontalschnitt durch Knochen-
                          und Prothesenschaft nahe dem proximalen
                          Schaftende,

Fig. 5                    eine der Fig. 1 entsprechende Teilansicht einer Prothese mit lösbarer Halsauflage

Fig. 6 und 7              eine Schnittansicht und eine Draufsicht auf die Halsauflage.

Fig. 8                    einen der Fig. 4 ähnlichen Schnitt durch
                          eine andere Ausführungsform und

Fig. 9 und 10             Teildarstellungen von Rippen mit unterschiedlichen Formen von Durchbrechungen.

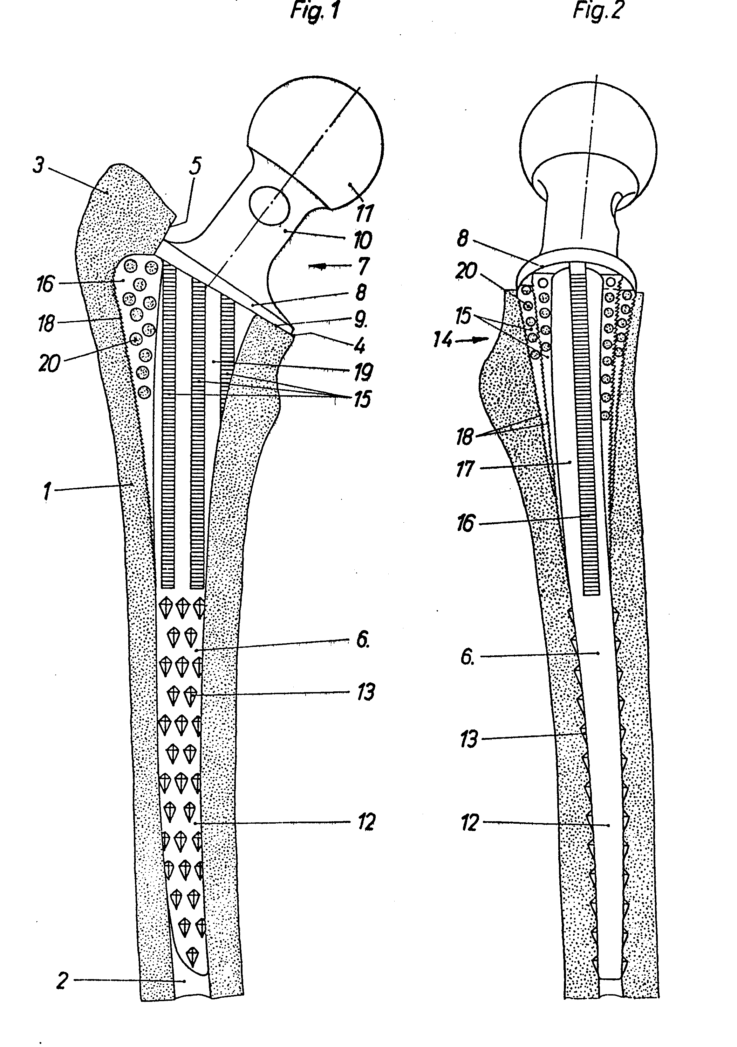

Die Schnittansichtsflächen des Oberschenkelknochens 1 sind in der Zeichnung punktiert angelegt. Man erkennt die Markhöhle 2, in Fig. 1 lateral den großen Trochanter 3 sowie die Resektionsflächen 4, 5, längs welchen der Oberschenkelhals mit dem Gelenkkopf entfernt ist.

In der ausgeräumten Markhöhle 2 befindet sich der zementlos eingesetzte Schaft 6 der Prothese 7. Der Schaft 6 wird proximal durch die Halsauflage 8 abgeschlossen, deren Unterfläche 9 auf der Resektionsfläche 4 aufliegt. Es schließt sich der Hals 10 mit dem Gelenkkopf 11 an. In den Ausführungsbeispielen der Fig. 1 bis 3 ist vorausgesetzt, daß alle diese Prothesenteile einstückig aus demselben Material, beispielsweise geschmiedetem Titan, bestehen.

Der Prothesenschaft 6 ist in seinem unteren Bereich 12 mit geschlossener Oberfläche ausgeführt, die zwecks besseren Verbundes mit dem Knochen mit Noppen 13 irgendeiner geeigneten Form versehen sein kann. In diesem Bereich ist die Markhöhle des Knochens vergleichsweise eng und individuell weniger unterschiedlich, so daß keine große Zahl variierender Prothesenformen erforderlich ist.

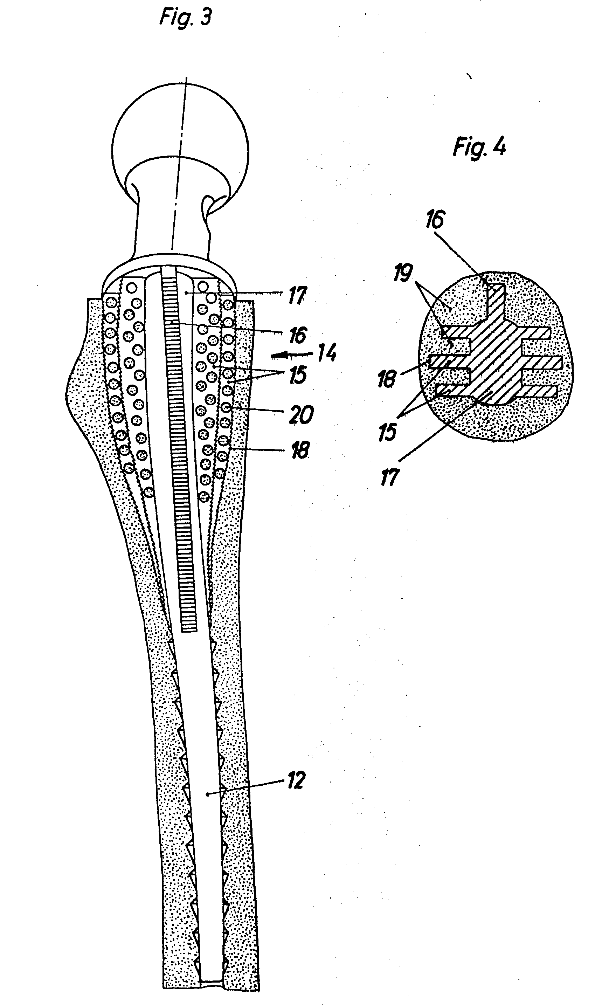

Am proximalen Ende erweitert sich die Markhöhle 2 in dem
Bereich, der allgemein mit der Bezugsziffer 14 angedeutet
ist, im allgemeinen trompetenartig wie dies in den Fig. 1
und 2 vorausgesetzt wurde. Es kommen jedoch auch andere
Erweiterungsformen vor, wie dies beispielsweise in Fig. 3
gezeigt ist. Um der Notwendigkeit zu entgehen, den Schaft 6
in diesem erweiterten Bereich massiv und in einer großen
Zahl von Formvariationen entsprechend der individuellen
Innengestalt der Corticalis zu gestalten, weist der
Prothesenschaft eine Mehrzahl von Rippen 15, 16 auf, die
von dem vergleichsweise dünnen Schaftkern 17 im Querschnitt weit nach außen vordringen und sich teilweise der
Corticalis nähern. Zumindest füllt der von den Rippen bestimmte Prothesenquerschnitt den spongiösen Teil des
trochanteren Femurbereichs großenteils aus. Die Tiefe der
Rippen (Hauptabmessung von ihrer Rückenfläche 18 bis zum
Schaftkern 17) ist am proximalen Ende am größten, während
sie distal allmählich bis auf Null verschwindet.

Im Querschnitt (Fig. 4) sind in den gegebenen Beispielen
sechs Rippen 15 auf gegenüberliegenden Seiten parallel zueinander angeordnet, während eine Rippe 16 quer dazu verläuft. Diese Anordnung gestattet die Herstellung durch
Schmieden. Wenn ein anderes Herstellungsverfahren gewählt
wird, können die Rippen auch in anderer Anordnung verlaufen. Wichtig ist jedoch stets, daß zumindest die am
weitesten medial gelegenen Rippen ausgeprägte, nach medial
gerichtete Kraftübertragungsflächen bilden. In einem
wesentlichen Teil ihrer Länge ist ihre Tiefenabmessung größer
als ihre Breite oder wenigstens etwa dieser gleich. Zwischen
den Rippen 15, 16 befinden sich Zwischenräume 19, in welchen
das vorhandene Knochengewebe verbleiben oder neues sich bilden kann. Die Rippen sind mit einer Vielzahl von Querbohrungen 20 versehen, die die Widerstandskraft der Rippen gegenüber Längskräften herabsetzen und diese dadurch nachgiebiger machen. Dieser Effekt wird in denjenigen Bereichen, in welchen die Rippen besonders tief sind, durch versetzte Anordnung der Durchbrechungen verstärkt. Außerdem kann Knochengewebe in die Durchbrechungen hineinwachsen und dadurch die Verankerung des Prothesenschafts im Knochen verbessern und auch die Übertragung von Zugkräften gestatten.

Die Rückenflächen 18 der Rippen sind gezahnt, was nicht nur dem formschlüssigen Verbund mit dem Knochengewebe dienen soll, sondern beim Einsetzen in den Röhrenknochen einen der Arbeitsweise einer Räumnadel gleichenden Effekt haben kann, durch den bei entsprechend knapper Vorbearbeitung des Knochenhohlraums gewährleistet wird, daß die Rückenflächen der Rippen ohne Zwischenraum am Knochengewebe anliegen.

Die Rippen können entsprechend der Richtung der Einsetzbewegung des Prothesenschafts in den Knochen gekrümmt sein, damit möglichst wenig Knochensubstanz vor dem Einsetzen bzw. während desselben weggeräumt werden muß.

Durch die ausgedehnten, mit dem Knochengewebe zusammenwirkende Seitenflanken der Rippen 15 wird die Kraftübertragung nach medial begünstigt. Die laterale Rippe 16, die in bekannter Anordnung in dem Trochanter eingreift, beteiligt auch diesen an der Kraftübertragung. Zusätzlich können weitere Mittel zur Übertragung von Kräften vom Prothesenschaft oder von der Halsauflage 8 auf die lateralen Bereiche des Knochens vorgesehen sein.

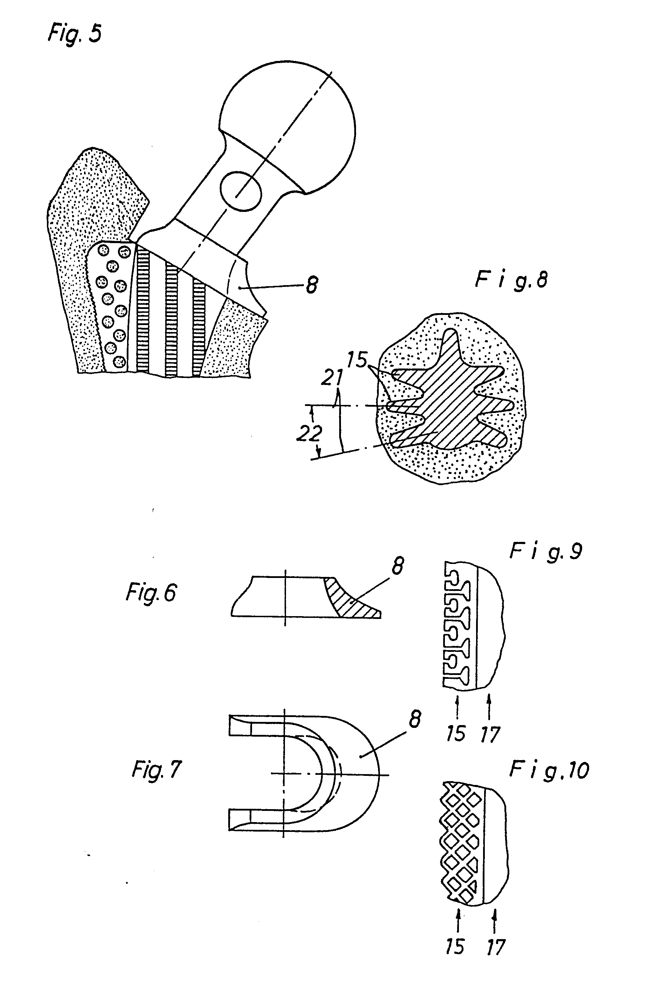

Die Längsnachgiebigkeit der Rippen kann durch seitliche
Einschnitte vergrößert werden. Beispielsweise ist in Fig. 9
vorgesehen, daß die Locher 20 sämtlich oder teilweise durch
einen Schlitz mit der Rippenoberfläche 18 verbunden sind. Dadurch wird das Widerstandsmoment des oberen Prothesenteils im
wesentlichen auf dasjenige des Schaftkerns 17 reduziert. An
den Außenflächen 18 der Rippen finden dann Kompressions- und
Dehnbewegungen statt, die für ein gegebenes Biegemoment denjenigen der natürlichen Knochensubstanz gleichen können.

Gemäß Fig. 10 ist vorgesehen, daß die Durchbrechungen wabenartig ausgebildet und angeordnet sind, derart daß sie von
dünnen, schräg zur Schaftlängesrichtung verlaufenden Stegen
getrennt sind, die so bemessen werden können, daß sich die
gewünschte Schaftelastizität ergibt.

Während die Halsauflage 8 in den Fig. 1 bis 3 einstückig mit
dem Prothesenschaft vorausgesetzt wurde, besteht sie im Fall
der Fig. 5 bis 7 aus einem besonderen, hufeisenförmigen Teil,
das nach dem Einsetzen der Prothese mit dieser auf nicht gezeigten Weise fest aber lösbar verbunden werden kann. Dies ermöglicht im Falle einer Reoperation leichteren Zugang zu den
Rippenzwischenräumen 19 zum Lösen des dortigen Verbundes mit
dem Knochengewebe.

Man erkennt, daß Prothesen mit unterschiedlicher Rippenform aus
gleichen Rohlingen durch entsprechend unterschiedliches Abfräsen der Rippenrücken erzeugt werden können. Zum Beispiel
können auch die Ausführungsbeispiele der Fig. 2 und 3 aus
gleichen Rohlingen erzeugt sein. Es stört nicht, wenn durch
den Materialabtrag von den Prothesenrücken einige der Duckbrechungen vollständig geöffnet werden, sofern nur andere
Durchbrechungen erhalten bleiben, die zum Rippenrücken im wesentlichen geschlossen sind und dadurch Zugkräfte zwischen
der Prothese und dem umgebenden Knochenmaterial durch Einwirkung auf das in sie eingewachsene Knochenmaterial übertragen können.

In Fig. 8 ist ein Prothesenquerschnitt gezeigt, in welchem die
Mittelachsen 21 benachbarter Rippen 15 einen engen Winkel 22
miteinander einschließen, der kleiner als 30° ist. Der zwischen
ihnen gebildete Raum 19 stellt - ebenso wie im Beispiel der
Fig. 4 - eine Kammer dar, in der Knochengewebe sich verankern
kann.

Als Vorteil der erfindungsgemäßen Prothese ist es zu werten,
daß die Rippenrücken 18, die sich nach der Operation im
unmittelbaren Kontakt mit Knochengewebe befinden sollen,
eine vorläufig ausreichende Kraftübertragung bei mäßiger
Belastung ermöglichen. Die volle Belastbarkeit stellt sich
mit dem Einwachsen des Knochens in die  Rippenzwischenräume 19
ein.

Die mittlere Weite der Zwischenräume sollte im allgemeinen
nicht kleiner als 2 mm sein. Als zweckmäßig hat sich eine
mittlere Weite von etwa 3 mm erwiesen.

Waldemar Link GmbH & Co
D/be

p 11747/85 EU

Patentanspruch

Femorale Hüftgelenkprothese mit einem zur zementfreien
Verankerung im Femur (1) geeigneten Schaft (6), der
eine Mehrzahl von von einem Schaftkern (17) vorspringenden
Längsrippen (15) aufweist, von denen die dorsal und ventral
vorgesehenen Rippen der medialen Seite eine Breitfläche
zukehren, dadurch gekennzeichnet, daß auf der dorsalen und
ventralen Seite des Schaftkerns (17) je eine Mehrzahl von
Längsrippen, die im Querschnitt einen Winkel von nicht mehr
als 30° miteinander einschließen, vorspringt, deren Tiefenabmessung ihrer Breite wenigstens etwa gleich ist.

1/5

Fig. 1

Fig. 2

0181586

*Fig. 3*

*Fig. 4*

Fig. 5

8

F i g.8

15

21

22

Fig. 6

8

F i g. 9

15  17

Fig. 7

8

F i g.10

15  17